Europäisches Patentamt

European Patent Office

Office européen des brevets

(11) Publication number: **0 131 166**
**B1**

# EUROPEAN PATENT SPECIFICATION

(45) Date of publication of patent specification: **07.09.88**

(51) Int. Cl.⁴: **A 61 M 1/00,** A 61 B 17/42,
A 61 M 25/00, A 61 M 31/00

(21) Application number: **84106732.5**

(22) Date of filing: **13.06.84**

(54) **Non-surgical apparatus for human embryo transfer.**

(30) Priority: **14.06.83 US 504308**
**14.06.83 US 504282**

(43) Date of publication of application:
**16.01.85 Bulletin 85/03**

(45) Publication of the grant of the patent:
**07.09.88 Bulletin 88/36**

(84) Designated Contracting States:
**AT BE CH DE FR GB IT LI LU NL SE**

(56) References cited:
**WO-A-82/00754**
**DE-A-3 106 306**
**US-A-3 506 010**
**US-A-3 527 203**
**US-A-3 777 743**
**US-A-3 889 657**
**US-A-4 004 588**
**US-A-4 178 936**

(73) Proprietor: **Fertility and Genetics Associates**
**135 South LaSalle Street**
**Chicago Illinois 60603 (US)**

(72) Inventor: **Seed, Richard G.**
**740 Belleforte**
**Oak Park Iliinois 60302 (US)**
Inventor: **Louw, John A.**
**31280 Calle Felicidad**
**Temecula California 92390 (US)**

(74) Representative: **Vossius & Partner**
**Siebertstrasse 4 P.O. Box 86 07 67**
**D-8000 München 86 (DE)**

Courier Press, Leamington Spa, England.

## Description

### Technical Field

This invention relates to an improved apparatus for use in non-surgical procedures for treating the human uterus. The apparatus of the invention, designated a uterine catheter, may be used for the non-surgical recovery step of methods for human embryo transfer or artificial embryonation, flushing or probing the uterus, introducing treatment materials into the uterus, or diagnostic procedures. The apparatus may in particular be used for non-surgical human embryo recovery in processes for human embryo transfer and artificial embryonation.

### Background of the Invention

In the United States, an estimated ten to fifteen percent of married couples are infertile. Infertility among about one-third of these couples is attributable to both partners. Fallopian tube disease afflicts approximately another one-third of the infertile couple population. In both situations, however, the infertile female usually has a hormonally responsive uterus capable of accepting an embryo and carrying it to term.

Both birth control and abortion have dramatically decreased the number of children now available for adoption by infertile couples. This decrease, in combination with the lack of effective fertility treatments has, up to now, forced many infertile couples to remain childless.

A detailed understanding of the interrelated hormonal processes that control the female reproductive cycle has been of particular importance in developing the methods of this invention.

The major anatomical elements involved in the reproductive cycle include the hypothalamus near the base of the brain, the pituitary gland just below the hypothalamus, the ovaries, the oviduct, the uterus and the vagina. In each turn of the normal human female reproduction cycle, a single oocyte is recruited and matures, is discharged from the ovary, and passes into the expanded end of the oviduct, where it then moves down to the uterus. In the absence of fertilization, the egg or oocyte disintegrates. This cycle is repeated at monthly intervals. If mating or insemination has occured at the right time in the cycle, the egg will meet sperm in the oviduct at the ampulla and may become fertilized.

The fertilized egg then moves slowly along the oviduct, nurtured and dividing as it goes. The egg usually reaches the uterus about three days later as a morula — a spherical cluster of 8 or more cells. The embryo begins to attach and to bury itself in the uterine wall within a period of from about the seventh through the tenth day after fertilization. Until then, it floats freely in the uterus from the day it enters the uterus until the moment of its attachment to the uterine wall.

The division of the cells continues in the uterus as the embryo evolves into a blastocyst, a fluid-filled spheric body with a polar cluster of cells, called the inner mass, at one side. The blastocyst hatches and attaches to the endometrium, the lining of the uterus and a complex implantation process embeds the blastocyst into the uterine wall. The inner cell mass becomes the fetus, whereas the outer cells of the blastocyst, together with neighboring uterine cells and blood vessels, form the placenta, the organ that provides for the exchange of materials between the fetal and the maternal circulation. With implantation, it may be said that the pregnancy is established, and is detectable by the measurement of hormones in the mother's blood, e.g., human chorionic gonodotropin (HCG).

The events surrounding conception and implantation are regulated by the interplay of hormones produced in the mother's hypothalamus, pituitary, ovaries, oviduct and uterus. Follicle-stimulating hormone (FSH) is secreted by the pituitary. FSH stimulates recruitment and maturation of the ovarian follicles which contain the oocyte and supporting cells. As the follicle grows, its cells secrete estrogenic hormones, chiefly estradiol, which act on the pituitary to reduce FSH, while simultaneously stimulating growth of the endometrium.

As the follicle approaches full size, FSH output decreases further and a second pituitary hormone, luteinizing hormone (LH), predominates. LH completes the maturation of the egg and triggers its discharge from the follicle into the oviduct. This discharge is believed to occur about one day after the time of the LH peak. The residual follicle cells left after the discharge of the oocyte form the corpus luteum, the anatomical structure which secretes progesterone, which induces further uterine wall development. If fertilizaton does not occur, the corpus luteum declines and a new cohort of ovarian follicles is recruited. The resulting decline in the estradiol/progesterone production precipitates degenerative changes in the uterine wall, and menstruation occurs.

After implantation into the uterine wall, the outer cells of the developing embryo secrete human chorionic gonadotropin (HCG), a hormone which functions much like LH. HCG stimulates continued maintenance and hormone production by the corpus luteum. HCG also acts to prevent the development of new follicles in the ovary. After approximately five weeks, the placenta takes over, secreting sufficient progesterone to maintain pregnancy.

One technique that has helped some infertile couples bear children is based on the surgical removal of an egg from the female, in vitro fertilization of that egg and the surgical return of the now-fertilized egg to the uterus of the female donor.

In vitro fertilization (IVF)/auto-transfer is a relatively new technique which is based on laparoscopic ovarian manipulation and surgical removal of a non-fertilized egg from preovulatory ovarian follicles of the infertile female, incubation of that egg, laboratory (in vitro) fertilization of that egg, a 2 to 3 day lab incubation of the fertilized egg and non-surgical replacement of the now fertilized egg back into the same woman's uterus.

External maintenance of the egg in such external fertilization has presented problems. The egg is normally suspended in complex bioligical fluids. Initially, there was little information concerning which constituents of these fluids were essential for continued health of the egg.

A similar problem was posed with the sperm, the other component of normal fertilization. In the initial ejaculate, the sperm are not prepared to penetrate the egg. They must first undergo capacitation, a process that is normally induced by conditions in the female genital tract. Capacitation must be simulated externally if the sperm are to be in the proper state to penetrate the egg.

Research on in vitro fertilization of non-human species, primarily marine animals, dates back to 1893. Two decades ago, convincing evidence was presented for the successful external fertilization of rabbit eggs. Since then, the process has been utilized in a number of other mammalian species, including humans.

The first human in vitro fertilization and attempted embryo transfer was reported by the English investigators R. G. Edwards, B. D. Bavister and P. C. Steptoe in 1969. The birth of Louise Joy Brown in July 25, 1978, was the first recorded case of in vitro fertilization/auto-transfer, in which a human egg had been surgically removed from a woman's ovary, fertilized in vitro and then implanted back into the same woman's uterus, where it developed to term. Such techniques do not allow for the non-surgical recovery of unfertilized eggs. They are complicated procedures with attendant surgical and anesthesia risks.

Embryo transfers or the recovery of an in vivo fertilized egg from a donor's uterus and its transfer into the uterus of another female, where the fertilized egg is allowed to implant and then to develop, has been performed in non-human species. The first such transfer was reported by .Hepe in 1890.

The largest number of embryo transfers have been made in cattle. However, successful embryo transfers or transplants have also been reported in other species of mammals including mice, rats, sheep, pigs, horses, rabbits, baboons, rhesus monkeys, and goats. *Egg Transfer in Cattle*, 1976, An Agricultural Research Seminar sponsored by the Commision of the European Communities, refers to some of these embryo transplant procedures. See also *Theriogenology*, published by Geron-X, Inc., Los Altos, California, *Science*, *Journal of Reproduction and Fertility*, and *The Biology of Reproduction*.

United States Patent No. 3,854,470 and *Bovine Embryo Transfer Procedures*, Colorado State University Animal Reproduction Laboratory, General Series 975 (1978, 1980) also refer to non-human embryo transplants, particularly as they relate to cattle. The '470 patent refers to the recovery of fertilized or non-fertilized ova in hooved mammals by means of a surgical recovery procedure, followed by non-surgical implantation using a transcervical cannula. United States Patent No. 4,326,505 .refers to a procedure for surgical implantation of embryos into host aniamls, particularly those having uterine horns. United States Patent Nos. 4,178,936 and 4,004,588 refer to methods for non-surgical ova recovery in animals, particularly cattle, using a catheter in combination with a rigid introducer.

Although embryo recovery and transfer has been practiced in cattle, as well as in some other non-human species, known techniques have heretofore been either impossible or impractical to effect in humans, becasue of basic anatomical and physiological differences between human and non-human mammalian female reproductive systems. A comparison of the bovine and human uterus reflects critical differences in, for example, size, shape and quality of tissue. In cattle, for example, the utero-tubal junction is tightly closed. Thus, fluids used in non-surgical bovine flushes are not lost out the oviducts. In humans, however, the opening from the uterus to oviducts is generally unobstructed. The use of bovine uterine flushing techniques and tools in a human subject results in loss of flushing media into the oviducts and peritoneal cavity. This may cause loss of the embryo or an ectopic pregnancy in the donor female.

Bovine non-surgical embryo recovery and non-surgical transfer methods also have involved painful displacement or repositioning of internal organs, thus requiring administration of regional or general anesthesia. The degree of anatomical control which can be effected in cattle is not possible in humans. Finally, steps employed in recovery and transfer processes within one species cannot, necessarily, be carried out in other species because of differences in size of reproductive organs, leakage of lavage fluid out of the oviducts, differences in reproductive cycles, as well as differences in recipient, donor or embryo responsiveness to drugs, compounds and media and the ineffectiveness or safety of specific recovery or transfer tools in different species.

Presently available apparatus designed for use in treating the human uterus are typically rigid devices. Such apparatus may cause discomfort to the patient and can lead to perforation of internal tissues. Additionally, the use of such rigid tools may require dilation of the cervix, repositioning of the uterus and use of clamps for stabilization. These procedures are often painful and can induce uterine bleeding.

United States Patent No. 3,527,203 refers to an apparatus for irrigating a body cavity which has an inlet tube, an outlet tube, and a sealing member. The tubes are made from a rigid plastic material, and have rough-edged apertures which are designed to facilitate the collection of tissue samples.

United States Patent No. 3,948,270 refers to a uterine cannula for human uterine elevation, which comprises a rigid tube positioned inside a Foley catheter. Due to its rigidity, this type of cannula cannot follow the natural contours of the uterus.

United States Patent No. 3,095,871 refers to a diagnostic tool for the human uterus. The tool comprises a hard rubber catheter, used in combination with a rigid stylet introducer.

Rigid uterine-invasive apparatus are also used in the field of animal husbandry. For example, United States Patent No. 3,854,470 refers to a rigid pipette containing a catheter for the insertion of ova into hooved mammals. United States Patent Nos. 4,178,936 and 4,004,588 refer to uterine catheters, used in combination with rigid introducers that penetrate the uterus for ova recovery, particularly in cattle.

A non-rigid catheter has been developed to facilitate non-surgical procedures for treating the human uterus. This apparatus causes virtually no discomfort to the patient and does not require the use of external introduction means or anesthesia. This apparatus combines stiffness and flexibility to good advantage in such non-surgical procedures.

Although this non-rigid catheter can be employed in the recovery of preimplantation embryos for human embryo transfer or artificial embryonation, it is somewhat disadvantaged in those applications because in operation it may lose lavage fluid out the oviducts and it may become obstructed by uterine tissue or mucus and, when obstructed may cause the loss of lavage fluid out the cervix. Any loss of such lavage fluid risks loss of the embryo. Moreover, when the fluid loss is through the oviducts an ectopic pregnancy may result. In addition, this catheter is not steerable within the uterine cavity, making it difficult to lavage the complete uterine surface. As a result, the recovery of preimplantation embryos is lower than desired using this catheter.

Because the preimplantation embryo recovery efficiency of that catheter in processes for non-surgical human embryo transfer and artificial embryonation is lower than desired, the full potential of those processes in treating human infertility, in avoiding genetic or hereditary diseases and in permitting prenatal adoptions has not been fully realized.

Summary of the Invention

The present invention is directed to an improved apparatus for use in non-surgical procedures for treating the human uterus. The apparatus of the invention, designated a uterine catheter, is of flexible, semi-rigid construction. It is characterized by ease of insertion without clogging with mucus or endometrial cells during entry through the cervix, the ability to lavage substantially all of the uterine surface, and a high percent recovery of the lavage fluid without clogging, thereby minimizing any loss of lavage fluid through the oviducts or cervix. Moreover, the recovered lavage fluid is usually clear and because it is free of blood and cellular debris it makes easier the location of any preimplantation embryo.

The uterine catheter of this invention may be used advantageously in non-surgical processes for human embryo transfer which permit the non-surgical recovery and the nonsurgical transfer of a fertilized egg from a human female donor to a human female recipient. Such processes broadly comprise the steps of synchronizing ovulation of both the donor and recipient, artificially inseminating the donor, non-surgically recovering the preimplantation embryo from the donor's uterus, locating the embryo in the lavage media and non-surgically transferring the embryo to the uterus of the recipient. More specifically, such methods comprise the steps of synchronizing ovulation of a human female donor and a human female recipient, artificially inseminating the donor, physically and non-surgically recovering the resulting preimplantation embryo from the uterus of the donor, locating the embryo in the recovery media and physically and non-surgically transferring the embryo into the uterus of the recipient.

Brief Description of the Drawings

Figure 1 is an elevational view of one embodiment of an improved double lumen catheter of the invention.

Figure 2 is an enlarged elevational view of forward distal end of the catheter of Figure 1.

General Desription of the Invention

Human embryo transfer, carried out using the uterine catheter according to the present invention, is a non-surgical, non-anesthetic, transvaginal and transcervical procedure for both the donor and the recipient.

The method of artifical embryonation, using the uterine catheter as described in this invention, is a non-surgical method for recovering a fertilized egg or preimplantation embryo from a human female donor and non-surgically transferring it to a human female recipient. Artificial embryonation is a method for recovering a preimplantation embryo from a human female whose egg has been fertilized by artificial insemination with semen from an infertile woman's husband and transferring of the resulting embryo to the uterus of the infertile woman.

The uterine catheter of the present invention is particularly useful in the recovery of pre-implantation embryos in processes for non-surgical human embryo transfer and artificial embryonation. In the processes of human embryo transfer and artificial embryonation, the uterine catheter of this invention is used as a flushing tool. It introduces and recovers lavage fluid containing the preimplantation embryo. Positive fluid pressure is used to introduce the lavage fluid into the uterus, and a negative pressure is used to remove this fluid and the embryo from the uterus.

The uterine catheter of the present invention can be used for a variety of non-surgical procedures for treating the uterus. For example, the uterine catheter can be used for probing the uterus, non-surgically recovering preimplantation embryos, flushing the uterus, testing for tubal patency, introducing a treating material into the uterus, and sampling endometrial or uterine tissue.

Alternatively, the improved catheter of this invention may be employed in a method for flushing the uterine cavity with a variety of solutions as a method of birth control.

The improved catheter of this invention facilitates non-surgical procedures for treating the uterus with minimal discomfort to the patient. The catheter is made of a semi-rigid, slippery material which permits it to move smoothly, but deliberately through the cervix, and it has sufficient flexibility to enable it to conform to uterine contours.

Methods for human embryo transfer using the uterine catheter of this invention begin with synchronization of ovulation of the donor and recipient to ensure similar uterine and hormonal environments for the embryo. Next, the donor is artificially inseminated using any of the methods known in the art. Most usually, the recipient's husband's semen is used. The fertilized egg or preimplantation embryo is then physically and non-surgically recovered from the uterus of the donor, located, and then physically and non-surgically transferred to the uterus of the recipient.

Non-surgical recovery of the fertilized egg or preimplantation embryo from the donor in accordance with this invention is accomplished by uterine lavage, carried out by insertion of a slender, curved, perforated instrument or catheter through the cervix into the uterus of the donor, who carries a fertilized egg that has not yet implanted itself into the uterine wall. Tissue culture media at body temperature is then flushed through the catheter into the uterus so as to flush the embryo out through the recovery channel of the instrument.

The embryo is then located in the culture media by use of a conventional light microscope. The embryo is then transferred to fresh media, washed and carefully examined under a high resolution microscope for fertilization, cell division, cell structure, and organization. Preferably, the ideal embryo to be transferred has developed to about the 50—100 cell stage. Transfer of embryos in a development stage below 50 cells is also contemplated by the method of this invention. However, it should be understood that implantation may be less efficient with embryos that have not matured to about 50 cells before transfer.

The embryo is then non-surgically transferred to the recipient. Transfer is accomplished by utilizing a non-surgical insertion catheter to insert the embryo through the cervix into the body of the uterus of the recipient. The embryo is then discharged into the uterus with a small amount of tissue culture fluid.

For the brief period that the embryo is outside of the donor's body, it is maintained in an environment that is as similar to the recipient's body as possible. This is accomplished by the use of incubators in which the humidity, acidity and vapor pressures of carbon dioxide and nitrogen are all carefully controlled. Preferably, the embryo is outside the body for no more than about 30 minutes, although longer times are acceptable. When longer times are used, the above-described incubation is preferably employed. Alternatively, the embryo may be cryo-preserved until it is transplanted into a recipient.

Human embryo transfer, including artificial embryonation, using the uterine catheter as described in this invention, is advantaged by being an office procedure for both donor and recipient. No surgery is performed on either the donor or the recipient. No anesthesia is needed and both donor and recipient tolerate the procedure well.

Artificial embryonation using the uterine catheter of the present invention is used when the donor is a fertile human female and the recipient is an infertile human female. The fertile donor is artificially inseminated with semen from the husband of the infertile recipient. The embryo is then transferred, by the methods of this invention, to the uterus of the infertile recipient. Although the resulting child does not carry any of the genes of the recipient, the child does carry the genes of both the husband and the donor. Moreover, the recipient carries the embryo for the entire gestation period, excluding a pre-transplant period of about five days.

Alternatively, the method using the catheter according to this invention permits embryo or prenatal adoption, by which a couple may adopt an embryo from another male and female. That embryo is then transplanted into the wife's uterus. In this case, the embryo carries the genes of neither the wife nor the husband, and fertility on the part of either husband or wife is not required.

Embryo transfer thus gives infertile women the opportunity to nurture, to share her bioligical rhythms with and to give birth to a child. Many consider this pregnancy and birth process a strong bonding experience which enables the recipient to fulfill the desire for her full measure of motherhood. Although the child does not carry the genes of the recipient, and is technically not genetically related to her, the birthing mother has carried and nurtured the child for subtantially the entire period of gestation and given birth to it.

Such methods may also be utilized in situations where the recipient has a family history of hereditary disease.

Human embryo transfer using the uterine catheter of the present invention can also be used in cases where a donor produces a viable embryo but cannot carry it to term. In this case, the embryo can be transferred to a female surrogate, who functions as a substitute mother to carry the embryo to term. This method is thus also useful for a donor with an incompetent cervix or a donor who because of disease or paralysis, is advised that a pregnancy would be dangerous or abnormal.

Advantageously, methods using the uterine catheter according to the present invention are non-surgical processes which do not usually require dilation of the cervic or clamping of the cervix with a tenaculum. Such procedures usually permit natural cervical positioning, rather than arbitrary cervical repositioning, and are therefore of minimal trauma and pain to both the donor and recipient.

In all of the above described procedures, the improved uterine catheter of the present invention can be inserted using the natural position of the cervix and uterus, without the use of clamps or any other type of positioning tool. The procedures are virtually painless to the patient being treated.

Embryo recovery through non-surgical flushing procedures using the uterine catheter of this invention, rather than a surgical procedure, is virtually painless and can, in many instances, be more effective than a surgical procedure. Such non-surgical, minimum-trauma embryo recovery procedures can be performed repeatedly on a given donor. It is possible, for example, to repeat the recovery procedure through several flushings of the donor's uterus over a period of up to three successive days, thus increasing the opportunity to make a successful embryo recovery.

The techniques described herein are believed to be especially useful and effective for the treatment of certain forms of human infertility without in vitro fertilization, and the recovery of assorted uterine and reproductive materials to enhance study of reproduction and medical diagnoses.

In addition, methods using the uterine catheter of this invention may be employed to recover embryos from a woman who chooses to bear children at some later data and stored in liquid nitrogen, in much the same way as sperm is stored in sperm banks. This "embryo bank" may be of particular use to women who want to postpone having children for professional reasons, or for women who, for reasons of convenience, prefer to save embryos in case they change their minds after having undergone a sterilization procedure.

Detailed Description of the Invention

It is clear that if implantation in a recipient of an externally fertilized embryo is to be successful, the maturation of the embryo must be closely matched to the state of the recipient's uterine wall. Synchronization of ovulatory cycles, as determined by the LH peak, the first step in the process of non-surgical human embryo transfer, provides the necessary hormonal match between donor and recipient in the normal events of ovulation, fetilization, oviductal transport, uterine maintenance, and implantation receptivity. The cycles of the donor and recipient are preferably synchronized to within plus or minus three days or less, most preferably to within plus or minus two days or less, with respect to time of ovulation. Synchronization is achieved by pharmacological methods to control ovulation dates or by utilizing pools of donors and recipients who are being tracked with hormonal measurements to select spontaneously ovulating matches. A detailed history of the menstrual cycle of each donor and recipient is, therefore, usually obtained.

Synchronization is preferably effected during the cycle prior to the cycle of conception, thereby advantageously providing a normal hormonal environment in which conception may occur. Preferably, by administering to donor, recipient or both, an effective amount of an oral estrogen-progesterone mixture (oral contraceptive) or vaginal progesterone suppositories (50 mg. once or twice daily), onset of menses is delayed and syncronization achieved. This treatment is started at least three days before the cyclic drop in basal body temperature. Menses will begin 24—36 hours after estrogen-progesterone withdrawal, and the ensuing cycle will be normal. Alternatively, a progestin is administered, for example, orally, topically, or parenterally, to either or both, the donor and recipient, in order to assure that the days of ovulation coincide. The support vehicle varies depending upon the route of administration, and known vehicles may be used. Synchronization can also, for example, be achieved or adjusted by administration to donor, recipient or both of effective amounts of a compound selected from the group consisting of progestins, estrogens, human chorionic gonadotropin (HCG), human menopausal gonadotropin (HMG), chlomiphene citrate, follicle stimulating hormone (FSH), gonadotropin releasing hormone (GNRH) and mixtures thereof in a suitable support vehicle. Alternatively, natural synchronization may be verified and appropriate donors and recipients matched.

After appropriate synchronization has been achieved, the embryo donor is artificially inseminated, for example, with semen from the husband of the infertile recipient. A deep cervical insemination technique is preferably used and insemination performed on the day of the LH peak, although insemination with ±1 day of the LH peak is also possible.

After fertilization, the embryo moves along the oviduct, dividing as it goes. The embryo usually reaches the uterus about three to four days after fertilization as a morula, a spherical cluster of eight or more cells. Cell division continues in the uterus to produce a blastocyst — a liquid-filled sphere with a group of cells, the inner cell mass, at one side. The bastocyst is surrounded by a clear, thick, gelatinous coating of proteinaceous material called the zona pellucida. The blastocyst "floats" freely in the uterus for several days. At approximately 7—9 days, the plus 100 cell embryo "hatches" from the zona pellucida. The hatched blastocyst embryo is then capable of implantation, which occurs within about 48 hours after hatching.

In methods using the uterine catheter of the invention, non-surgical embryo recovery involves uterine lavage of the preimplantation embryo into the recovery channel or suction lumen of an instrument or catheter. Uterine lavage of the donor's uterus is preferably carried out within about 3 to 7 days after the LH peak. Because the emmryo has not yet been implanted, the lavage can recover the embryo. It can then be transferred to the recipient in its normal preimplantation stage.

The improved uterine catheter of this invention comprises (a) at least two lumens at the forward distal end defined by an external tube and at least one internal tube, the tubes being coaxial to each other; (b) a forward distal end adapted for insertion into the human cervical canal and endometrial cavity and having a closed rounded tip, said tip being adjacent to the forward distal end of the inner tube(s); (c) a cage attached distally to said closed tip and proximally to the distal end of the external tube, the cage surrounding the distal most end of the inner tube(s) which are located coaxially therein, said distal most end of the inner tube(s) having means for delivering lavage fluid to the uterine cavity; (d) a rear proximal end, said end being adapted to permit separate connection of the inner and outer tubes to a fluid supply and a recovery reservoir; said catheter preferably having an exterior diameter between about 2.3 to 5.3 mm (7 and 16 French) and having a combination of flexibility and stiffness sufficient to allow the catheter to describe a 10° to 30° angle per six centimeters when inserted into the uterine cavity to conform to uterine contours and to be steerable within the uterine cavity. The catheter is of flexible, semi-rigid construction in order to follow undisturbed human contours and to allow conformation with various shapes, sizes, positions and movements found in human uterine configurations.

The catheter may be of materials such as polyvinyl chloride, polyethylene, or Teflon® (tetrafluoroethylene). The preferred catheter is transparent in order to permit observation of fluid contents and motion while the uterine catheter is in use. It may also be desirably coated with Teflon®, a silicone, or a similar slippery resin.

The uterine catheter of the present invention has a small diameter which permits ready positioning through the cervical os and into the uterus. The catheter is preferably a two lumen device, one lumen to introduce a lavage fluid into the uterus and the other lumen to remove the lavage fluid and embryo from the uterus. The exterior diameter of the catheter is preferably between about 7 French (2.3 mm) to 16 French (5.3 mm) and more preferably 11 French (3.6 mm). The uterine catheter is generally about 30 cm in length.

The relative flow of liquid through the lumens of the catheter at working pressures is important. The supply lumen(s) must not provide fluid at a rate substantially greater than that removed by the suction lumen(s). Neither should the suction lumen(s) recover fluid at a rate substantially greater than that supplied by the supply lumen(s). Preferably, the rates of supply and recovery are substantially the same. To accomplish this equalization of supply and recovery, we regulate the pressure of the liquid flow and the size of the lumens. In a preferred embodiment of this invention, the flow is adjusted to accommodate a ratio of diameters of suction lumen to supply lumen of between 5:1 and 15:1.

The tubes which define the lumens should be made of resilient, flexible, smooth material, e.g., Teflon® (tetrafluoroethylene), polyvinylchloride and polyethylene. The inner surface of the suction lumen defined by the inner and outer tubes should be slippery enough to permit uninterrupted fluid flow and to avoid loss of embryos due to sticking to the tube surfaces. This may be accomplished by treating the tube walls with silicone sprays or by other methods known in the art. It should be understood that any catheter construction having at least two lumens is within the scope of this invention so long as at the forward distal end of the catheter the lumens are defined by an external tube and at least one internal tube.

The closed rounded tip is hard. It may be constructed of high impact plastic or resin or metal. It should be rounded, and preferably is bullet shaped. In one preferred embodiment of this invention, the tip is attached to the forward distal end of the inner tube.

The cage may be of any design or material which is sufficient to protect the space defined by the cage from obstruction by mucus and/or endometrium during treatment of the uterine cavity with liquid and the recovery of that liquid. For example, the cage may be of a helical or ribbed design and be constructed of a preferably non-corrosive, rigid material, such as stainless steel.

In a preferred embodiment of this invention, the means for delivering lavage fluid to the uterine cavity are radial spray holes in the distal most end of the inner tube protected by the cage. These radial spray holes have a diameter of between about 0.003 and 0.020 inches (.08 to 0.5 mm). While they may be located randomly along the distal most end of the inner tube, we prefer to locate them adjacent the point where the distal end of the inner tube is connected to the rounded tip. In that location we believe the uterine lavage and embryo recovery is most effective. The holes are located in such a manner as to prevent uterine lavage spray from hitting the structural members that define the cage.

The catheter has a combination of flexibility and stiffness sufficient to describe a 10° to 30° angle per six centimeters when inserted into the uterine cavity, to allow it to conform to the uterine contours and to be steerable within the uterine cavity. We prefer to use catheters for recovery of preimplantation embryos whose flexibility and stiffness are defined as two inches (51 mm) of catheter clamped on one end deflecting at the free end 0.2 inches (5 mm) under loads ranging from 0.5 to 2.5 oz (.015 to .075 kg). We also prefer to use catheters that describe a 10° to 30° angle, and more preferably a 15° angle, per six centimeters before insertion into the uterine cavity.

The improved catheter of this invention may be used both with, and without, an accompanying guide or catheter sleeve. Preferably, when the catheter is used for embryo recovery, a guide is employed. The guide has four major functions: (1) to stabilize the catheter during its application to the cervix; (2) to provide a shield for the coiled spring tip of the catheter during insertion through the cervix, thereby preventing the tip from becoming clogged with cervical secretions; (3) to gauge easily uterine depth, therby preventing potentially damaging deep penetration of the catheter into the uterine cavity; and (4) to transmit rotary motion to the catheter, allowing the operator to sweep substantially all of the uterine surface.

The optional guide or catheter sleeve is about 8 to 17 French (2.6 to 5.6 mm) in external diameter. It is made of a rigid material, e.g., copper, brass, stainless steel or rigid plastic. It is about 10 to 14 cm in length. The guide also has a locator flange positioned about 2 to 2.5 cm from the forward distil end of the guide. The flange, which is usually perpendicular to the sleeve, is positioned so as to come to rest on the exocervix such that the guide extends only 2 to 2.5 cm into the endocervical canal and does not enter the uterus.

The catheter is inserted into the guide such that the guide is positioned externally and coaxially to the catheter. The fit between the guide and catheter must be tight enough to prevent lavage fluid loss from the uterine cavity between the sleeve and the catheter. Accordingly, there is a snug fit with essentially no space between the catheter and sleeve. However, the catheter may be slid freely back and forth inside the sleeve. The guide is characterized by a locator flange so that when inserted into the endocervical canal, the guide extends no more than between about 2 to 2.5 cm at an angulation of about 0 to 30°. Accordingly, the sleeve or guide never enters the uterus.

Ready entry of the catheter and guide of this invention into the endocervical canal is facilitated because (1) the uterine catheter has a blunt, preferably bullet-shaped, nose and (2) the guide provides a segmented rigidity to the catheter that is useful during insertion.

Alternatively, it is possible to use a flushing tool without a guide or sleeve which has sufficient stiffness that a 4 cm length will resist, without bending, a vertically applied weight of up to 15 grams; and a 4 cm length will bend under the application of a horizontally applied weight of 10 grams at a distance of 1.0 to 3.0 cm. The exterior surface of such tool is slippery, with a coefficient of friction between 0.03 and 0.15. Such a tool penetrates the human uterus without the need for accessory introduction means and yet will conform to the various sizes, shapes and irregularities characterizing a given human female reproductive system.

The flushing tool may also include an inflatable cuff for retention and blockage of flow of the flushing fluid. In use, the tool does not irrigate the uterus constantly. The uterus is temporarily sealed with a small balloon, inflated with the flushing fluid, and the liquid is subsequently removed using negative pressure.

In operation the improved catheter of this invention provides a fluid under positive pressure from a supply source to the uterine cavity. The positive pressure is preferably provided either by a syringe or a gravity feed. The catheter also allows recovery of substantially all of that fluid from the uterine cavity under negative pressure or gravity. The negative pressure is preferably caused by a motorized pump.

An advantageous feature of the method using the catheter of the invention is that multiple flushes of the donor's uterus to recover an embryo are possible, because the procedure is virtually painless and the donor is not reluctant to undergo the flushing procedure more than once. This permits flushing, for example, over a period of up to three successive days, in order to ensure recovery of a viable embryo.

Flushing is preferably carried out using an intravenous stand. For example, a flushing fluid container or fluid controller is placed on the stand, with tubes connecting the flushing tool or catheter and the container, and the height of the container adjusted up or down to obtain the desired fluid column pressure. Adjustment of the height of the container relative to the subject thus controls the pressure of the fluid entering the catheter. A one meter adjustment provides approximately an additional 1/15th of atmospheric pressure to the column of fluid. The outlet may also be used to vary pressure, for example, by the use of a syringe or motorized vacuum pump, to give negative pressure to the outlet fluid. A combination of these procedures is preferably used. Alternatively, the fluid may be delivered by means of an assistant holding a hand-held syringe.

Control of inlet and outlet pressure combined with the suction of the uterine wall over the spiral tip may eliminate the risk of tubal pregnancy which could result from a reverse flush of the embryo into the oviduct.

Tissue culture liquid raised to body temperature is normally used for lavage. The fertilized egg or embryo is washed out in the fluid in the suction channel of the lavage tool. Typically, the lavage fluid volume ranges between about 5 and 150 ml, preferably between 30 and 60 ml, for each procedure. Most preferably, uterine lavage of the donor's uterus is carried out by supplying 60 ml of lavage fluid over a period of about 5 minutes.

The fluid used may be one of several suitable for tissue culture. Examples include Hams F—10, Dulbecco's phosphate buffered saline (PBS), described in the *Journal of Experimental Medicine*, 98:167 (1954) as an aqueous solution of calcium chloride, potassium chloride, potassium dihydrogen phosphate, magnesium chloride, sodium chloride, and sodium hydrogen phosphate; and Gibco Medium 199 or TCM—199, described in *Proceedings of the Society of Experimental Biology and Medicine*, 73:1 (1950) as an aqueous solution of calcium chloride, ferric nitrate, potassium chloride, potassium dihydrogen phosphate, magnesium sulfate, sodium chloride, sodium bicarbonate, and sodium hydrogen phosphate. These media should be buffered to pH 7.0 to 7.5 and preferably in the range of pH 7.3 to 7.4.

Additives to the basic tissue culture medium may include human serum albumin (up to 20 percent), antibiotics, antimycotics, and energy sources, such as glucose, and vitamins.

After the embryo is flushed from the uterus of the donor, it is located by microscopic examination of the liquid culture fluid. In contrast to cow or mouse embryos, for which the greater numbers of transplants have been done, a human embryo is more difficult to find, because it is nearly transparent. After the embryo is located, it is transferred to and washed with fresh media and carefully examined under a high resolution microscope for fertilization, division, cell structure and organization. A preferred embryonic developmental stage for transfer according to this invention is the 50—100 cell stage.

After location and examination, the embryo is picked up in a transfer catheter for insertion into the uterus of the recipient. As described above, the embryo is maintained externally for a period preferably less than 30 minutes, although longer times are acceptable.

If the embryo is to be maintained externally for over 30 minutes, it is held in an incubator in which the controlled atmospheric environment preferably contains between about 0.5 and 5 percent $CO_2$, up to 95 percent relative humidity, between about 5 and 15 percent oxygen, balanced nitrogen, and physioligical temperature to avoid temperature shock on entry and removal.

Transfer of the embryo is accomplished by insertion of the embryo into the uterus of the recipient, utilizing a non-surgical insertion catheter or an insertion tool in which the embryo is contained in a 5 to 50 µl volume of tissue culture fluid. Such insertion catheters include a Tomcat catheter and other conventional insertion cervical catheters. This instrument is inserted by the vagina and through the cervix and into the body of the uterus where the embryo is discharged with a small amount of tissue culture fluid. The catheter may be inserted into the dome of the uterus and the embryo delivered through either a side-ported or open-tipped catheter. Alternatively, the catheter may be passed 2 cm, or less, above the internal os of the cervix.

As stated above, the recovered embryo may also be frozen for storage over an extended period before thawing and transfer. Problems in freezing and storage of embryos involve the mechanical and biochemical destruction of cells by the expansion of water upon freezing. Therefore, it is essential to use cryo-protective agents which perform several functions: (1) reduce the amount of water in the cell without killing it; (2) reduce the expansion upon freezing; (3) develop uniformly distributed micro-crystals instead of a few large macro-crystals; and (4) are not poisonous in and of themselves.

Freezing is done slowly to permit cellular accommodation. A final solidification often does not occur until −60 or 80°C, with many cryo-protective agents and cellular constituents. These conditions can be satisfied in whole or in part by using stepwise serial concentrations of cryo-protective agents prior to freezing and in conjunction with temperaturte lowering, and inversely, using stepwise serial dilution of cryo-protective agents upon thawing and warming. Cryo-protective agents include DMSO (dimethyl sulfoxide), and glycerol.

The non-surgical embryo recovery may also be used as a means for preventing unwanted pregnancies in humans. Fluid is introduced and the embryo flushed and recovered before implantation, thus preventing implantation and pregnancy. Preferably, the fluid contains an effective amount of embryocidal agents, selected from the group consisting of estrogens (estradiol-17 beta), hyperosmotic compounds, hypertonic glucose, propylene glycol, glucose, prostaglandins, acids, alkalis and urea, and mixtures thereof. These agents are normally mixed with normal saline for flushing. This process is used on the third or fourth day after sexual contact, when an unwanted conception could have occurred, and may be repeated on one or more of three successive days.

Description of the preferred embodiments

Referring now to Figures·1 and 2, we have shown in elevational view one embodiment of an improved double lumen catheter (1) of this invention.

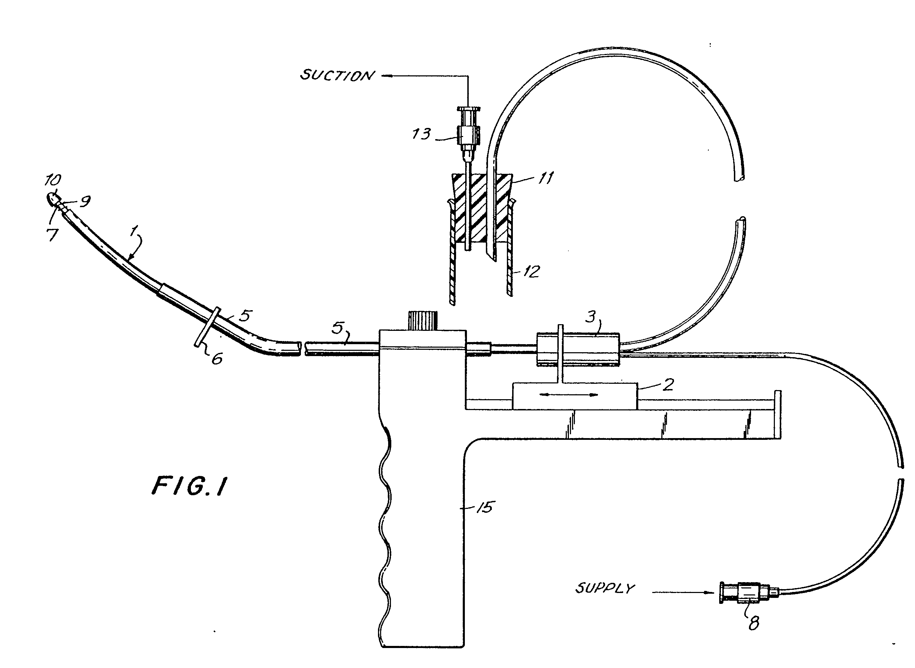

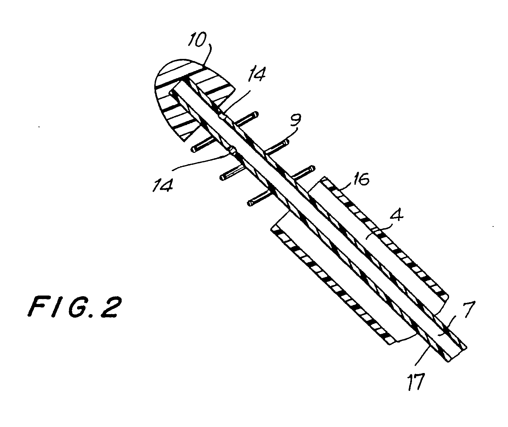

As shown in Figures 1 and 2, the catheter (1) comprises two lumens (4 and 7 in Figure 2) defined by an external tube (16) and an internal tube (17), the tubes being coaxial to each other. The forward distal end of catheter (1) is adapted for insertion into the endocervical canal and endometrial cavity. It has a closed, rounded tip (10), the tip being attached to the forward distal end of the inner tube (17). The catheter (1) also comprises a cage (9) attached distilly to the rounded tip (10) and proximally to the distal end of the external tube (16). The cage surrounds the distal end of the inner tube (17), which is coaxially located within it. The distal most end of the inner tube (17) has a number of radial spray holes (14) located in such a manner so as to prevent uterine lavage spray from hitting the structure members that define the cage.

The catheter also has a rear proximal end adapted to permit the separate connection of inner tube (17) and outer tube (16) to a fluid supply and recovery reservoir (12).

The catheter depicted in Figure 1 has been inserted into a metal sleeve or guide (5) which is characterized by a locator flange (6) which is perpendicularly positioned on the sleeve so as to come to rest on the exocervix. In that position the guide extends no more than between about 2 to

$2\frac{1}{2}$ centimeters into the endocervical canal. The guide does not enter the uterus. As shown in Figure 1, the guide also has an angulation between 0 to 30° to enable steering of the catheter within the uterus by rotation.

As shown in Figure 1, the catheter also has a handle (15) and a slide (2) and manifold (3) which allow easy determination of the depth of insertion of the catheter into the utrine cavity and permit separate connection of the inner and outer tubes (16 and 17) to a fluid supply and reservoir (12). The catheter shown in Figure 1 also includes a number of fittings (e.g., 8 and 11) which permit connection with the tubes separately to those supply and recovery vessels. The embodiment of Figure 1 also permits the supply of fluid under positive pressure to the uterine cavity through holes (14) and the removal of substantially all of that fluid under negative pressure to the recovery vessel (12).

As shown in Figures 1 and 2, the cage (9) in the embodiment depicted is defined by the helical spring-like structure located around the distal most end of the inner tube (17). As illustrated in Figure 2, the holes (14) and the structural members defining the cage (9) are located relative to each other such that the lavage spray from holes (14) does not hit the structure members.

### Examples

The following examples demonstrate the method of human embryo transfer using the uterine catheter according to the invention.

These examples represent the results of twelve attempts to recover embryos from donors (4—5 donors) whose ovulation has been synchronized with that of the recipient, either naturally or artificially (using oral contraceptives), and who had been artificially inseminated with the recipient's husband's semen. In these processes, three embroyos have been recovered (Examples 1, 2 and 3), three transfers to the recipient's uterus have been made (Examples 1, 2 and 3) and one pregnancy has resulted (Example 2).

### Example 1

Example 1 demonstrates the non-surgical recovery of a fertilized egg or embryo from the uterus of a donor in an artificial embryonation method. It also demonstrates the location of that embryo in the lavage fluid and its non-surgical transfer into the uterus of the recipient. However, no evidence for embryo implantation or pregnancy was obtained.

This lack of pregnancy is not unexpected statistically because about 30—50% of natural fertilizations result in spontaneous abortions. Moreover, the relative immaturity (14 cells) of the recovered embryo in this example may have also contributed to the failed pregnancy in this example.

In this example, the recipient was a 23-year-old woman whose two previous pregnancies had been unsuccessful (one spontaneous abortion and one ectopic). She has a $3\frac{1}{2}$ year history of infertility, related to complications of appendicitis and pelvic infections. She had also undergone four laparoscopies and four laparotomies. Her left tube and ovary were absent, while the right adnexae were involved extensively with adhesions. She had regular ovulatory cycles. The recipient's husband was a 35-year-old man in excellent health.

The donor was a 26-year-old woman who has had three previous pregnancies (all terminated by voluntary abortions). The donor has regular ovulatory cycles.

The recipient, her husband, and the donor were evaluated by detailed medical histories, physical examinations, laboratory screening, psychological testing and interviews with clinical psychologists. Pedigree analysis and karyotypes were done on the husband and donor. The donor and recipient were also matched for phenotypic features and major blood groups, although this is not necessary in the process of this invention. All parties has signed individual informed consent forms and the project had been approved by a review board.

The donor and recipient were matched to have natural ovulatory synchronization on identical days so that no external synchronization was necessary. It should, however, be understood that such external or artificial synchronization may be achieved by retarding or advancing the cycles of the donor, recipient or both, as previously described. Both donor and recipient began showing simultaneous preovulatory rises in estradiol on the same day (donor-240 pg/ml; recipient-235 pg/ml) and has simultaneous luteinizing hormone (LH) peaks (donor: >100 mIU/ml; recipient: 98 mIU/ml), three days later.

The donor was artificially inseminated by conventional means into the cervix on the same day as her LH peak with a 3.0 cc semen specimen from the recipient's husband (semen analysis count $114 \times 10^6$/cc, with good motility). No sperm was present in the donor's cervical mucus prior to insemination.

Five days after insemination, the donor's uterus was washed with 60 ml tissue culture fluid (Hams F—10), supplemented with 5% human serum albumin and glucose to recover the embryo. The uterine lavage was done using a semi-rigid catheter having a guiding means as previously described. Over 98% of the lavage solution was recovered. The donor tolerated the procedure well. No anesthesia was used.

The recovered fluid was then examined under a microscope and a 14 cel embryo identified. The embryo was washed with additional media, incubated, and transferred transcervically with a side-ported catheter into the uterus of the recipient. The transfer took place $2\frac{1}{2}$ h after lavage. The recipient remained supine on the examining table to 2 h and tolerated the procedure well. No anesthesia was used.

The donor experienced the onset of normal mentrual flow 2 weeks after her LH peak. The recipient has a tense $4 \times 4$ cm right overian mass and began spotting intermittently 9 days after LH peak. However, serial serum concentrations of human chorionic gonadotropin (HCG) remained less than 5.0 mIU/ml. Menses in the recipient began 13 days after the LH peak.

### Example 2

Example 2 demonstrates the non-surgical recovery of a fertilized egg or embryo from the uterus of a donor in an artificial embryonation method. It also demonstrates the location of that embryo in the lavage fluid, its transfer into the uterus of the recipient and the implantation of the embryo into the uterine wall of the recipient. That pregnancy is now in its ninth week.

In this example, substantially the same procedure as Example 1 was followed. However, in this example, the recipient's LH peak occurred 2 days before the donor's LH peak (the same donor as in Example 1).

Again, the donor was artificially inseminated with the recipient's husband's semen (2.5 cc; $33 \times 10^6$/cc with good motility) on the day of peak LH (66 mIU/ml).

The donor's uterus was lavaged 5 days after artificial insemination, substantially as described in Example 1, using 60 cc of the same tissue culture solution. Again, recovery of the lavage fluid was substantially complete. Again, the recovery was tolerated well by the donor.

The embroyo (~50 cells) was located in the lavage fluid, washed with additional media, incubated, and transferred to the recipient using a conventinal Tomcat catheter in media supplemented with 70% cord serum. The transfer took place 1.5 h after lavage. Again, the recipient remained supine on the examining table for 2 h after transfer. Again, the transfer was tolerated well by the recipient.

Twenty days after transfer, the recipient's uterus was soft and slightly enlarged and her HCG was elevated. Thirty-three days after transfer, the recipient has a well developed uterine sac, as demonstrated by echoes in ultrasound examination. Fetal heart was not yet detectable. The recipient coplained of nausea. For seven weeks after transfer the recipient is still complaining of nausea, but has no other complications.

### Example 3

This example demonstrates the non-surgical recovery of a fertilized egg or embryo from the uterus of a donor in an artificial embryonation method. It also demonstrates the location of that embryo in the lavage fluid and its transfer into the uterus of the recipient. However, no embryo implantation or pregnancy was observed.

Again, the lack of implantation and pregnancy is not an expected statistically (*supra*). Moreover, the relative immaturity of the recovered embryo (6 cells) may have also contributed to the failed pregnancy in this example.

Again, the procedure used in this example is substantially the same as the one employed in Examples 1 and 2. In this example, the donor's LH peak occurred naturally one day before that of the recipient. The donor was artificially inseminated with 3.0 cc semen from recipient's husband one day after her LH peak and her uterus was lavaged, as before, five days later. A six cell embryo was located in the lavage fluid and transferred (3 h after lavage) with a Tomcat catheter into the uterus of the recipient. Again, both donor and recipient tolerated the procedure well, although the recipient had mild cramping. The recipient has resumed menses.

Having described the invention with particular reference to the preferred form thereof, it will be apparent to those skilled in the art to which the invention pertains . after understanding the invention, that various changes and modifictions may be made therein without departing from the scope of the invention as defined by the claims appended hereto.

### Claims

1. An uterine catheter (1) which comprises (a) at least two lumens (4, 7) at the forward distal end defined by an external tube (16) and at least one internal tube (17), the tubes being coaxial to each other; (b) a forward distal end adapted for insertion into the human cervical canal and endometrial cavity and having a closed, rounded tip (10), said tip being adjacent to the forward distal end of the internal tube(s); (c) a cage (9) attached distally to said closed tip and proximally to the distal end of the external tube, the cage surrounding the distal most end of the internal tube(s) which are located coaxially therein, said distal most end of the internal tube(s) having a means (14) for delivering lavage fluid to the uterine cavity; (d) a rear proximal end, said end being adapted to permit separate connection of the internal and external tubes to a fluid supply and a recovery reservoir (12); said catheter having an exterior diameter of about 2.3 to 5.3 mm (7 to 16 French). and having a combination of flexibility and stiffness sufficient to allow the catheter to describe a 10° to 30° angle per six centimeters when inserted into the uterine cavity, to conform to uterine contours and to be steerable within the uterine cavity.

2. The catheter according to claim 1, wherein there are two lumens defined by one external and one internal tube.

3. The catheter according to claim 1 or 2, wherein the tip is attached to the forward distal end of the internal tube(s).

4. The catheter according to any of claims 1 to 3, wherein the distal most end of the internal tube(s) has a number of radial spray holes having a diameter of between .08 to 0.5 mm (0.003 and 0.020 inches).

5. The catheter according to any of claims 1 to 4, wherein said catheter is curved before insertion into said uterine cavity, said curve describing a 15° angle per six centimeters.

6. The catheter according to any of claims 1 to 5, wherein said catheter is curved before insertion into said uterine cavity, said curve describing a 10° to 30° angle per six centimeters.

7. The catheter according to any of claims 1 to 6, having an external diameter of 3.6 mm (11 French).

8. The catheter according to any of claims 1 to 7, made of a material which is polyvinyl chloride, polyethylene or tetrafluoroethylene.

9. The catheter according to any of claims 1 to 8, which is transparent.

10. The catheter according to any of claims 1 to 9, which is about 30 cm in length.

11. The catheter according to any of claims 1 to 10, wherein the tubes defining the lumens are made of a material which is tetrafluoroethylene, polyvinyl chloride or polyethylene.

12. The catheter according to any of claims 4 to 11, wherein the radial spray holes are located adjacent to the point where the distal end of the internal tube is connected to the rounded tip.

13. The catheter according to any of claims 1 to 12, having a flexibility and stiffness defined as 51 mm (two inches) of catheter clamped on one end deflecting at the free end 5 mm (0.2 inches) at loads ranging from .015 to .075 kg (0.5 to 2.5 ounces).

14. A combination of the improved catheter of claims 1 and a rigid sleeve, said catheter being inserted snugly into said sleeve such that the sleeve is positioned externally and coaxially to the catheter, the sleeve being characterized by a locator flange that is positioned to come to rest on the exocervix so that the sleeve can extend no more than between about 2 to 2.5 cm into the endocervical canal.

15. The combination of catheter and sleeve according to claim 14, wherein said sleeve has an external diameter of about 2.6 to 5.6 mm (8 to 17 French).

16. The combination of catheter and sleeve according to claim 14 or 15, wherein said sleeve has a length of about 10 to 14 cm.

17. The combination of catheter and sleeve according to any of claims 14 to 16, wherein said sleeve has an angulation of about 0 to 30°.

**Patentansprüche**

1. Intrauterin-Katheter (1) umfassend
(a) mindestens zwei Bohrungen (4, 7) am vorderen freien Ende, die durch ein äußeres Rohr (16) und mindestens ein inners Rohr (17) gebildet werden, die zueinander koadial sind;
(b) ein zur Einführung in den menschlichen Cervix-Kanal und in die Endometrium-Höhle angepaßtes vorderes Freies Ende mit einer geschlossenen, abgerundeten Spitze (10), die an das vordere freie Ende des (der) inneren Rohres (Rohre) angrenzt;
(c) ein Gehäuse (9), das abgewandt von der geschlossenen Spitze und in der Nähe des freien Endes des äußeren Rohres befestigt ist, wobei das Gehäuse das äußerste freie Ende des (der) inneren Rohres (Rohre) umgibt, die koaxial darin angeordnet sind, wobei das äußerste freie Ende des (der) inneren Rohres (Rohre) eine Einrichtung (14) zur Freisetzung einer Spülflüssigkeit in die Uterushöhle aufweist;
(d) ein hinteres Ende, das so ausgebildet ist, daß es die getrennte Verbindung der inneren und äußeren Rohre mit einer Flüssigkeitszufuhr und einem Wiederauffangbehälter (12) gestattet; wobei der Katheter einen Außendurchmesser von etwa 2,3 bis 5,3 mm (7 bis 16 French) hat und eine ausreichende Kombination von Flexibilität und Steifheit aufweist, die es ihm erlaubt, beim Einführen in die Uterushöhle einen Winkel von 10° bis 30° pro 6 cm zu beschreiben, so daß er sich an die Uteruskonturen anpaßt und innerhalb der Uterushöhle steuerbar ist.

2. Katheter nach Anspruch 1, bei dem durch ein äußeres und ein inneres Rohr zwei Bohrungen gebildet werden.

3. Katheter nach Anspruch 1 oder 2, bei dem die Spitze am vorderen freien Ende des (der) inneren Rohres (Rohre) befestigt ist.

4. Katheter nach einem der Ansprüche 1 bis 3, bei dem das äußerste freie Ende des (der) inneren Rohres (Rohre) mehrere in radialer Richtung weisende Sprühöffnungen mit einem Durchmesser von 0,08 bis 0.5 mm (0.003 bis 0,020 Inches) aufweist.

5. Katheter nach einem der Ansprüche 1 bis 4, der vor dem Einführen in die Uterushöhle gebogen ist, wobei die Biegung einem Winkel von 15° pro 6 cm beschreibt.

6. Katheter nach einem der Ansprüche 1 bis 5, der vor dem Einführen, in die Uterushöhle gebogen ist, wobei die Biegung einen Winkel von 10 bis 30° pro 6 cm beschreibt.

7. Katheter nach einem der Ansprüche 1 bis 6 mit einem Außendurchmesser von 3,6 mm (11 French).

8. Katheter nach einem der Ansprüche 1 bis 7, der aus Polyvinylchlorid, Polyäthylen oder Tetrafluoräthylen gefertigt ist.

9. Katheter nach einem der Ansprüche 1 bis 8, der transparent ist.

10. Katheter nach einem der Ansprüche 1 bis 9, der eine Länge von etwa 30 cm aufweist.

11. Katheter nach einem der Ansprüche 1 bis 10, bei dem die die Bohrungen bildenden Rohre aus Tetrafluoroäthylen, Polyvinylchlorid oder Polyäthylen gefertigt sind.

12. Katheter nach einem der Ansprüche 4 bis 11, bei dem sich die in radialer Richtung weisenden Sprühöffnungen neben der Stelle befinden, an der das freie Ende des inneren Rohres mit der abgerundeten Spitze verbunden ist.

13. Katheter nach einem der Ansprüche 1 bis 12 mit einer Flexibilität und Steifheit, die dadurch definiert ist, daß sich ein an einem Ende festgeklemmter Katheter mit einer Länge von 51 mm (2 Inches) am freien Ende bei einer Belastung mit 0,015 bis 0,075 kg (0,5 bis 2,5 Unzen) um 5 mm (0,2 Inches) verbiegt.

14. Kombination eines verbesserten Katheters nach Anspruch 1 und einer festen Manschette, wobei der Katheter in die Manschette dicht eingepaßt ist, so daß die Manschette außen und koaxial zum Katheter liegt, dadurch gekennzeichnet, daß die Manschette einen Schnellverbindungsflansch aufweist, der so angeordnet ist, daß er außerhalb der Cervix liegt, so daß sich die Manschette nicht weiter als etwa 2 bis 2,5 cm in den Endocervixkanal erstreckt.

15. Kombination von Katheter und Manschette nach Anspuch 14, wobei die Manschette einen Außendurchmesser von etwa 2,6 bis 5,6 mm (8 bis 17 French) aufweist.

16. Kombination von Katheter und Manschette nach Anspruch 14 oder 15, wobei die Manschette eine Länge von etwa 10 bis 14 cm hat.

17. Kombination von Katheter und Manschette nach einem der Ansprüche 14 bis 16, wobei die Manschette etwa 0 bis 30° abgewinkelt ist.

## Revendications

1. Cathéter utérin (1) qui comprend (a) au moins deux passages (4, 7) à l'extrémité distale antérieure définis par un tube extérieure (16) et au moins un tube intérieure (17), les tubes ayant le même axe; (b) une extrémité distale antérieure destinée à être insérée dans le canal cervical humain et la cavité utérine et comportant un bout arrondi fermé (10), ce bout étant contigu à l'extrémité distale antérieure du (des) tube(s) interne(s); (c) une cage (9) fixée distalement au bout fermé et proximalement à l'extrémité distale du tube extérieur, la cage entourant l'extrémité la plus distale du (des) tube(s) interne(s) qui sont situés coaxia-lement dans son intérieur, l'extrémité la plus distale du (des) tube(s) interne(s) comportant un moyen (14) pour fournir un fluide de lavage à la cavité utérine; (d) une extrémité proximale postérieure, cette extrémité étant destiné à permettre une connexion séparée des tubes interne et externe avec une alimentation en fluide et un réservoir de récupération (12), le cathéter ayant un diamètre extérieur d'environ 2,3 à 5,3 mm (numéros 7 à 16 de la charrière française) et présentant une combinaison de flexibilité et de rigidité suffisante pour permettre au cathéter de décrire un angle de 10 à 30° par six centimètres lorsqu'il est inséré dans la cavité utérine, d'épouser les contours utérins et de pouvoir être dirigé à l'intérieur de la cavité utérine.

2. Cathéter selon la revendication 1, dans lequel il y a deux passages définis par un tube extérieur et un tube intérieur.

3. Cathéter selon la revendication 1 ou la revendication 2, dans lequel le bout est fixé à l'extrémité distale antérieure du (des) tube(s) interne(s).

4. Cathéter selon l'une quelconque des revendications 1 à 3, dans lequel l'extrémité la plus distale du (des) tube(s) interne(s) a un nombre de trous radiaux de pulvérisation ayant un diamètre compris entre 0,08 et 0,5 mm.

5. Cathéter selon l'une quelconque des revendi-dictions 1 à 4, dans lequel le cathéter est incurvé avant insertion dans la cavité utérine, la courbe décrivant un angle de 15° par six centimètres.

6. Cathéter selon l'une quelconque des revendications 1 à 5, dans lequel le cathéter est incurvé avant insertion dans la cavité utérine, la courbe décrivant un angle de 10 à 30° par six centimètres.

7. Cathéter selon l'une quelconque des revendications 1 à 6, ayant un diamètre extérieur de 3,6 mm (numéro 11 de la charrière française).

8. Cathéter selon l'une quelconque des revendications 1 à 7, constitué d'une matériau qui est du chlorure de polyvinyle, du polyéthylène ou du tétrafluoroéthylène.

9. Cathéter selon l'une quelconque des revendications 1 à 8, qui est transparent.

10. Cathéter selon l'une quelconque des reven-dictions 1 à 9, qui a environ 30 cm de long.

11. Cathéter selon l'une quelconque des revendications 1 à 10, dans lequel les tubes définissant les passages sont constitués d'un matériau qui est le tétrafluoroéthylène, le chlorure de polyvinyle ou le polyéthylène.

12. Cathéter selon l'une quelconque des revendications 4 à 11, dans lequel les trous radicaux de pulvérisation sont situés près du point où l'extrémité distale du tube interne est connectée au bout arrondi.

13. Cathéter selon l'une quelconque des revendications 1 à 12, ayant une flexibilité et une rigidité définies comme correspondant à 51mm du cathéter fixé sur une extrémité déviant à l'extrémité libre de 5 mm pour des charges comprises entre 0,015 et 0,075 kg.

14. Combinaison du cathéter perfectionné de la revendication 1 et d'un manchon rigide, le cathéter étant inséré par ajustage à frottement doux dans le manchon de sorte que le manchon est placé à l'extérieur et coaxial'ement au cathéter, le manchon étant caractérisé par un rebord de positonnement situé de mainère à venir reposer sur l'éxocol de sorte que le manchon ne peut pas s'étendre de plus d'environ 2—2,5 cm dans l'en-docol canal.

15. Combinaison d'un cathéter et d'un manchon selon la revendication 14, dans laquelle la manchon a un diamètre extérieure d'environ 2,6 à 5,6 mm (numéro 8 à 17 de la charrière française).

16. Combinaison d'un cathéter et d'un manchon selon la revendication 14 ou la revendicaton 15, dans laquelle le manchon a une longueur d'environ 10 à 14 cm.

17. Combinaison d'une cathéter et d'un manchon selon l'une quelconque des revendications 14 à 16, dans laquelle le manchon a une forme angulaire d'environ 0 à 30°.

SUCTION

SUPPLY

FIG.1

0 131 166

FIG. 2